# DEMANDE DE BREVET EUROPEEN

(11) **EP 1 516 631 A1**
(43) Date de publication de la demande: **23.03.2005**
(21) Numéro de dépôt: 03292639.6
(22) Date de dépôt: 22.10.2003
(51) Int. Cl.: A61K 47/44

(54) **Onguent, pour le corps à effets décongestionnant et faire disparaitre progressivement les douleurs musculaires, foulures, entorses, déboitements, courbatures et apaiser les douleurs articulaires et rhumatismales**

(30) Priorité: 24.10.2002 FR 0213328
(71) Demandeur: Aliaga, Maria Yolanda, 75015 Paris (FR)
(72) Inventeur: Aliaga, Maria Yolanda, 75015 Paris (FR)

(57) **Abrégé**

Cette invention concerne un onguent sous forme d'huile et liniments pour le corps à effets décongestionnant et faire disparaitre progressivement les douleurs musculaires, foulures, entorses, déboitements, courbatures et apaiser les douleurs articulaires et rhumatismales. L'efficacité de cet onguent réside dans le choix minutieux de ses composants et dans leur dosage personnalisé.

La simplicité d'emploi de l'onguent le destine à l'usage du grand public. Cet onguent est caractérisé en ce qu'il contient de la noix de muscade, de l'acide salicylique, beurre de karité et des huiles essentielles.

## Description

La présente invention concerne un onguent sous forme d'huile et liniments conçu pour soulager et faire disparaitre progressivement les douleurs musculaires, foulures, entorses, déboitements, décongestionner et apaiser les douleurs articulaires et rhumatismales.

L'onguent sous forme d'huile et liniments, par un apport local d'éléments appropriés tels que: acide salicylique, noix de muscade ainsi que les huiles essentielles au pouvoir de pénétration des couches de la peau, lui confèrent, par leur richesse en acides gras, une pléiade de qualités. Certains phénols sont anti-inflammatoires et bloquent le cycle-oxygénase déclencheur de l'inflammation.
- Contre la raideur musculaire due parfois à un effort prolongé ou occasionnel, les névralgies articulaires, douleurs rhumatismales, fatigue et impression de faiblesse des membres inférieurs.
- Sa grande éfficacité due à un choix et à un dosage juidicieux de ses composants, à une concentration et une diffusibilité des huiles essentielles permettant des résultats rapides et satisfaisants.
- Ses propriétés régénérantes diminuent l'amplitude des contractions musculaires: raideurs du cou, torticolis, névralgie cervicale, faisant ainsi retrouver bien être et élasticité.
- L'onguent satisfait à ces éxigences par son pouvoir apaisant, décongestionnant, doux et sans aucun effet traumatisant.
- Sa simplicité d'emploi: quelques minutes suffisent chaque jour à son application.

La qualité des HE que vous utilisez est de première importance. Les HE de mauvaise qualité ne donneront pas les résultats escomptés.

### UTILISATION

Sur une peau nettoyée et sèche, réchauffer les parties atteintes. EXEMPLE DOS: avec une serviette chaude frictionner la partie du corps endolorie; ensuite, faire pénétrer l'onguent par effleurages (2 à 3 fois par jour, selon l'empleur de la douleur) en insistant sur les parties atteintes.

DOULEUR LOCALISEE: Avec l'onguent, imbiber une compresse (ou un pansement américain) et l'apposer sur la partie endolorie.

FOULURE : frictionner doucement le ligament douloureux.

### RESULTATS

Dés la première application on éprouve un bien être qui s'amplifie de jour en jour.

La durée du traitement varie selon la nature de la douleur, de son ancienneté et de l'effet recherché.

Cet onguent a été testé sur des humains.

On trouvera ci-dessous quelques éxemples de composition uniquement dans un but de précision et non de limitation de la portée de l'invention.

### EXEMPLE I

| | | | | |
|---|---|---|---|---|
| Noix de muscade (graine) | 2 graines | 1 1/2 graine | 1 graine | 1 graine |
| Acide salicylique | 5 grammes | 3 grammes | 2 gr. | 1 graine |
| Huile de maïs | 60 ml. | 50 ml. | 25 ml. | 20 ml. |
| Huiles essentielles : | | | | |
| - Anis | 5 gttes. | 4 gttes | 2 gttes | 1 gtte |
| - Basilique éxotique | 5 " | 4 " | 2 " | 1 " |
| - Camomille matricaire | 5 " | 4 " | 2 " | 1 " |
| - Camphre | 5 " | 4 " | 2 " | 1 " |
| - Cajeput | 5 " | 4 " | 2 " | 1 " |
| - Citronelle | 5 " | 4 " | 2 " | 1 " |
| - Estragon | 5 " | 4 " | 2 " | 1 " |
| - Eucalyptus | 5 " | 4 " | 2 " | 1 " |
| - Hysope | 5 " | 4 " | 2 " | 1 " |
| - Marjolaine | 5 " | 4 " | 2 " | 1 " |
| - Arnica (teinture) | 5 " | 4 " | 2 " | 1 " |
| - Mélisse | 5 " | 4 " | 2 " | 1 " |
| - Pin sylvestre ou écosse | 5 " | 4 " | 2 " | 1 " |
| - Romarin à cinéole | 5 " | 4 " | 2 " | 1 " |
| - Souci | 5 " | 4 " | 2 " | 1 " |
| - Thym | 5 " | 4 " | 2 " | 1 " |
| - Ylang-ylang | 5 " | 4 " | 2 " | 1 " |
| - Genièvre | 5 " | 4 " | 2 " | 1 " |

### EXEMPLE DE PREPARATION :

Râper (broyer) la noix de muscade et macérer avec l'huile de mais pendan un mois environ, décanter et filtrer puis ajouter l'acide salicylique; mélanger bien le tout en ajoutant les huiles essentielles.

Conditionner immédiatement dans un flacon de teinte foncée et dont le couvercle est à vis.

Entreposer à l'abri des rayons solaires.

Dans le cas de préparations simultanées, des précautions toutes particulières sont à prendre pour éviter confusion et contamination.

A titre d'exemple on peut utiliser:
huile de pépin de raisin
huile de soja
HE Fenouil doux, Eucalyptus citronné, Epinette noire,
Curcuma,

### EXEMPLE 2

- Vaseline : 20 gr.
- Huile de noix de muscade : 10
- Huile de maïs : 10
- Borate de sodium : 0,2 gr.
   Camphre : 0,1 gr.

### HE

Hélichryse italienne : 2 gttes; Vétivier : 1 gtte; Anis : 1 gtte,
Gauthéries : 2 gttes; Laurier : 2 gttes; Bouleau : 1 gtte;
Romarin : 1 gtte; Cannelle de Ceylan : 2 gttes; Menthe : 2 gttes;
Eucalyptus : 2 gttes.

### PREPARATION

Dans un bol en verre ou en inox, déposer la vaseline, ajouter l'huile de noix de muscade, le borate de sodiume préalablement dissous, ajouter le camphre, ensuite les huiles essentielles tout en mélangeant le tout jusqu'à l'obtention d'une homogénéité parfaite.

### EXEMPLE 3

- Beurre de karité : 40 gr.
- Huile de noix de muscade : 15 gr.
- Huile de maïs : 25 gr; Camphre : 0,1 gr.
- Ethanol : 10 ml.

### HE

Calendula : 2 gttes; gengembre : 1 gtte; Jowan : 2 gttes; Inule : 1 gtte.
Cyprès : 2 gttes; Camomille : 3 gttes; Romarin à cinéole : 3 gttes.
Sauge : 3 gttes; Géranium : 2 gttes; Clous de girofle : 5 gttes; Cumin
des prés : 2 gttes; Origan : 2 gttes.

### PREPARATION

Dissoudre le beurre de karité au bain marie.

Une fois dissous y ajouter l'huile de maïs, l'huile de noix de muscade l'Ethanol ainsi que les huiles essentielles tout en remuant le tout.

L'objet de l'invention ayant été décrit, il est déclaré que ce qui constitue l'essentiel de l'invention est ce qui est concrétisé dans les revendications qui suivent.

## Revendications

1. Onguent pour le corps **caractérisé en ce qu'**il contient :
- huiles essentielles,
- noix de muscade,
- acide salicylique,

2. Utilisation de l'onguent selon la revendication n° I pour préparer un médicament destiné à soulager et faire disparaitre progressivement les douleurs musculaires, courbatures, foulures et apaiser les douleurs articulaires et rhumatismales, **caractérisé en ce qu'**il contient :
| | | |
|---|---|---|
| Noix de muscade (graine) | 2 graines | 1 graine |
| Acide salicylique | 5 grammes | 1 gramme |
| Huile de maïs | 60 ml | 20 ml |
| | | |
| Huiles essentielles : | | |
| Anis | 5 gttes | 1 gtte |
| Basilique éxotique | 5 " | 1 " |
| Camomille matricaire | 5 " | 1 " |
| Camphre | 5 " | 1 " |
| Cajeput | 5 " | 1 " |
| Citronelle | 5 " | 1 " |
| Estragon | 5 " | 1 " |
| Eucalyptus | 5 " | 1 " |
| Hysope | 5 " | 1 " |
| Marjolaine | 5 " | 1 " |
| Arnica (teinture) | 5 " | 1 " |
| Mélisse | 5 " | 1 " |
| Pin sylvestre ou écosse | 5 " | 1 " |
| Romarin à cinéole | 5 " | 1 " |
| Souci | 5 " | 1 " |
| Thym | 5 " | 1 " |
| Ylang-Ylang | 5 " | 1 " |
| Genièvre | 5 " | 1 " |
Vaseline : 20 gr.
Huile de noix de muscade : 10 ml
Huile de maïs : 10 Ml
Borate de sodium : 0,2 gr.
Camphre :0,1 gr.
HE
Hélichryse italienne : 2 gttes,
Vétivier : 1 gtte,
Anis: 1 gtte,
Gauthéries : 2 gttes,
Laurier : 2 gttes,
Bouleau : 1 gtte;
Romarin : 1 gtte,
Cannelle de Ceylan : 2 gttes,
Menthe : 2 gttes,
Eucalyptus : 2 gttes.
